# EUROPEAN PATENT SPECIFICATION

(11) **EP 1 282 408 B1**
(45) Date of publication and mention of the grant of the patent: **21.07.2010**
(21) Application number: 01977953.7
(22) Date of filing: 15.05.2001
(51) Int. Cl.: A61K 9/70, A61F 13/02, A61L 15/16, A61M 37/00

(54) **HYDROGEL COMPOSITION FOR TRANSDERMAL DRUG DELIVERY**
HYDROGELZUSAMMENSETZUNG ZUR TRANSDERMALEN ARZNEISTOFFABGABE
COMPOSITION D'HYDROGEL POUR ADMINISTRATION TRANSDERMIQUE DE MEDICAMENTS

(30) Priority: 16.05.2000 KR 2000026091
(43) Date of publication of application: 12.02.2003
(73) Proprietor: Samyang Corporation, Chongno-ku, Seoul 110-470 (KR)
(72) Inventor: KIM, Ho Chin, Yusung-ku, Daejeon 305-717 (KR); YOON, Hye Jeong, Daejeon 302-190 (KR)
(74) Representative: Smyth, Gyles Darren
(86) International application number: PCT/KR2001/000783
(87) International publication number: WO 2001/087276

(56) References cited:
- EP-A1- 0 904 778
- WO-A1-93/00058
- WO-A1-95/18603
- WO-A2-99/15210

## Description

### FIELD OF THE INVENTION

The present invention relates to a hydrogel composition for transdermal drug delivery and more specifically, to a hydrogel composition for transdermal drug delivery comprising a hydrophilic polymer base, a drug, a lipophilic permeation enhancer and a compatibilizer consisting essentially of an acrylate polymer which compatibilizes the lipophilic component, i.e. the enhancer, with the hydrophilic polymer base and which renders a uniform composition which is thermodynamically stable. The acrylate polymers such as acrylic acid polymers, methacrylic acid polymers, alkyl acrylate polymers, alkyl methacrylate polymers or copolymers thereof function as compatibilizers in this invention which enables both the hydrophilic and lipophilic components to be uniformly mixed in a hydrogel composition thereby providing for effective drug delivery.

### BACKGROUND OF THE INVENTION

Transdermal drug delivery has many advantages over oral or injection means for administering drugs into the body such as efficiency and easiness of control of drug release and administration. Transdermal delivery of various drugs is well known in the art of drug delivery. However, not all drugs can be applied as a transdermal drug delivery system because most of the drugs cannot effectively penetrate the skin. Therefore, the skin-penetration of drugs has to be increased by altering the physical and chemical properties of the skin keratotic layer or subcutaneous fat layer by decreasing the diffusional resistance through reversible damage or increasing the solubility of the drugs in the skin in order to obtain enough skin-penetration for the drug to be effective. Additives performing these actions can be collectively referred to as permeation enhancers.

Generally, a polymer base is used for the transdermal delivery of drugs, as a solvent for the drugs and the skin permeation enhancers. A polymer base should have sufficient mechanical strength, elasticity and adhesion to skin to be used as transdermal base. Much research has been undertaken to obtain these physical properties. For example, Okabe discloses a polymer base containing a water-soluble preparation, polyacrylamide gel. A gel including multivalent metal salt in polyacrylic acid or its salt [Japanese Patent Publication No. 3-167117], and gel including monomers having sulfonic acid groups [Japanese Patent Publication No. 4-91021] are also known. However, those water-soluble preparations are easily dissociated and since most of proteins have positive or negative charges at above or below their isoelectric point, there is a problem of the drug being bonded to the dissociated preparation.

Hydrogel patches for transdermal delivery of drugs are also known in the art. These patches typically include an inert, impervious backing layer, an adhesive layer containing a polymer base and the drug, optional selected excipients, and a release liner that is peeled off and discarded before applying the patch to the skin. Suitable polymer bases may be one or more members selected from the group consisting of polyvinyl alcohol and polyvinyl pyrrolidone, maleic anhydride/vinyl ether copolymer, gelatin, alginate, hydroxyethyl methacrylate, cargeenane, hydroxyethyl cellulose, silicone rubber, agar, hydroxypropyl cellulose, hydroxypropylmethyl cellulose, methyl cellulose, carboxyvinyl copolymer, polyethylene oxide, polyethylene glycol, polyacryl amide, polyhydroxyethyl methacrylate, polydioxolane, polyacrylic acid, polyacryl acetate, polyacryl amide and polyvinyl chloride may be used. Preferred are polyvinyl alcohol, polyvinyl pyrrolidone, maleic anhydride/vinyl ether copolymer and hydroxyethyl cellulose. The drug and selected excipients, if any, are directly incorporated into the hydrophilic polymer solution and then mixed to give a hydrogel composition containing the drug and excipients. See, for example, Makoto Haga et al., Lecture Summary of the 112th Conference of The Pharmaceutical Society of Japan, 4, 52(1992)], Riviere J. E. et al., J. Pharm. Sci., 81(6), 504(1992), Banga A. K. et al., Pharm. Res., 10(5), 697(1993), Japanese Patent Publication No. 3-193057. However, these gels exhibit poor adhesion to skin.

Japanese Patent Publication No. 5-230313 discloses a gel obtained by mixing highly water-absorptive or hydrophilic polymer with polyvinyl alcohol. Though this gel has sufficient adhesion, it has poor mechanical strength and thus it is difficult to form the gel. There have been attempts to increase the crosslinking density by adding a crosslinking agent, like glutaldehyde, or by irradiation in order to increase the mechanical strength. Although this method improves the mechanical strength to some degree, the water content and adhesiveness of the gel decrease so that it is no longer suitable to be used as transdermal polymer base.

US Patent No. 4,593,053 discloses a hydrophilic gel matrix comprising a polar plasticizer and a hydrophilic gel matrix of polyvinyl pyrrolidone and polyvinyl alcohol. US Patent No. 5,082,663 discloses a water-soluble polymer gel of carboxymethyl cellulose including moisturizers like glycerol, sorbitol, propylene glycol and 1,3-butanediol. However, since these polymer gel matrices are water-soluble or hydrophilic, the permeation enhancers and the drugs used are limited to those that are water-soluble or hydrophilic. Since the skin layer like the keratotic layer or the subcutaneous fat, which functions as the greatest penetration barrier for most of drugs, is lipophilic or sub-lipophilic, it is well known that a sufficient penetration rate cannot be obtained with hydrophilic or water-soluble permeation enhancer only. In addition, most of conventional hydrogel compostions suffer the drawback of being unstable with respect to the water and humectant included therein. In other words, these preparations tend to synerese, i.e. to exclude the liquid, water component of the gel.

Accordingly, a technique of preparing an uniform, stable, hydrogel composition for transdermal drug delivery, which includes both a hydrophilic component, i.e. a hydrophilic polymer base, and lipophilic substances, i.e. permeation enhancers, is needed.

WO 99/13812 discloses transdermal dosage forms for MENT. Typical gel formulations comprise methyl cellulose, carbapol, benzyl alcohol, propylene glycol, isopropyl alcohol, ethanol and water.

WO 96/27372 discloses a water-based topical cream containing nitroglycerin, a penetration enhancer, water, a thickener, and an emulsifier. Example 2 discloses the following composition:

| | |
|---|---|
| Water | 70.75% |
| Nitroglycerin | 1.00 |
| Propylene Glycol | 15.00 |
| Glycerine | 5.00 |
| Laurocapram | 1.50 |
| Isopropyl Palmitate | 1.00 |
| Methylcellulose | 1.80 |
| Carbomer 934P | 1.05 |
| Brij 78 | 1.50 |
| Triethanolamine | 1.00 |
| Sodium Borate | 0.25 |
| Butylated Hydroxytoluene | 0.05 |
| Methyl Parabens | 0.05 |
| Propyl Parabens | 0.05 |

EP 0 812 587 discloses transdermal compositions containing Nimesulide. Example 1 discloses the following topical gel dosage form:

| Component | Quantity |
|---|---|
| Nimesulide | 2.0 g |
| Dimethylacetamide | 22.0g |
| Ethyl Alcohol | 40.0 g |
| Acetone | 10.0g |
| Cremophor RH 40 | 4.0 g |
| Propylene glycol | 38.0 g |
| Polyethylene glycol 400 | 48.8 g |
| Carbopol 934 | 4.0 g |
| Water | 30.0 g |
| Diethylamine | 1.2 g |
| Total | 200.0 g |

### SUMMARY OF THE INVENTION

The present invention provides a stable hydrophilic polymer preparation which includes both hydrophilic and lipophilic substances as permeation enhances in order to effectively deliver drugs transdermally. Briefly, in one aspect, the invention relates to an improved transdermal drug delivery composition comprising a hydrophilic polymer base, a drug, a lipophilic permeation enhancer, water as a solvent and a compatibilizer consisting essentially of an acrylate polymer which compatibilizes the lipophilic component, i. e. the enhancer, with the hydrophilic polymer base and which renders a uniform composition which is thermodynamically stable,
wherein said acrylate polymer is a copolymer comprising a 1:2 ratio of methyl methacrylate and ethyl acrylate expressed by the following Formula (1a), 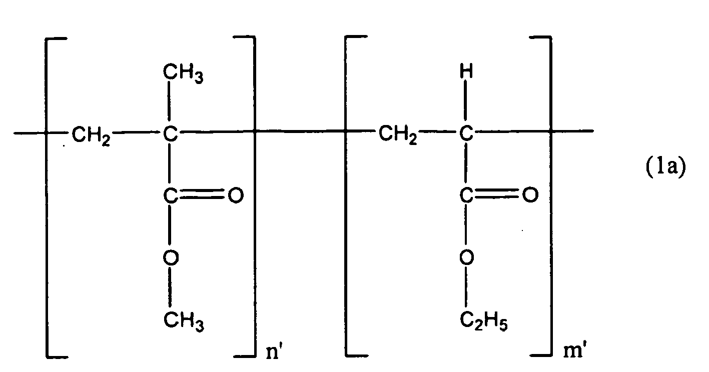 wherein the ratio of n' to m' is 1:2, n' is an integer between 200 to 10,000 and m' is an integer between 400 to 20,000 or a copolymer comprising a 1:1 ratio of methacrylic acid and ethyl acrylate expressed by the following Formula (1b), 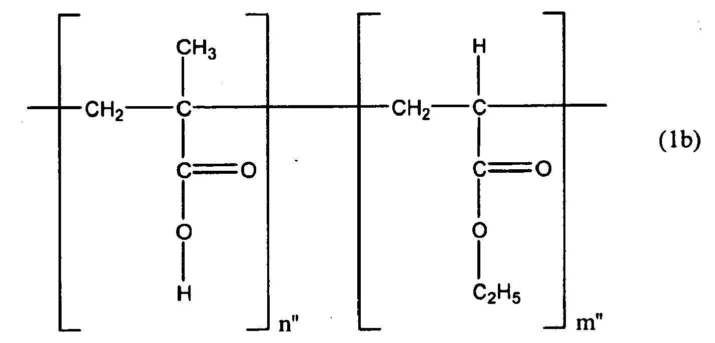 wherein the ratio of n" to m" is 1:1 and n" and m" is an integer between 300 to 10,000, and wherein a mixture of polyvinyl alcohol and polyvinyl pyrrolidone is comprised in the composition as the hydrophylic polymer in the range of 2-30 wt% of polyvinyl alcohol and 2-20 wt% of polyvinyl pyrrolidone based on the weight of the transdermal hydrogel composition.

The hydrophilic polymer base affects the mechanical strength, elasticity and adhesive properties and can comprise one or more hydrophilic polymers selected from the group consisting of polyvinyl alcohol, polyvinyl pyrrolidone, maleic anhydride/vinyl ether copolymer, gelatin, alginate, hydroxyethyl methacrylate, carrageenan, hydroxyethyl cellulose, silicone rubber, agar, hydroxypropyl cellulose, hydroxypropylmethyl cellulose, methyl cellulose, carboxyvinyl copolymer, polyethylene oxide, polyethylene glycol, polyacryl amide, polyhydroxyethyl methacrylate, polydioxolane, polyacrylic acid, polyacryl acetate, polyacryl amide and polyvinyl chloride. The acrylate polymer used as the compatibilizer is a copolymer comprising a 1: 2 ratio of methyl methacrylate and ethyl acrylate or a copolymer comprising a 1:1 ratio of methacrylic acid and ethyl acrylate. The content of the acrylate polymer used as the compatibilizer in the present invention is preferably within the range of 0.1-10wt. %, and is preferably 2-8wt. %, of the entire composition.

Herein are also described methods of preparing an improved stable hydrogel composition for transdermal drug delivery which contains both a hydrophilic polymer base and lipophilic penetration enhancers. Also described is a method to compatibilize a hydrophilic polymer base and lipophilic components in order to make a drug delivery preparation that is uniform and stable, thus overcoming the problems exhibited by current preparations.

### Brief Description of the Drawings

FIG. 1 is a schematic diagram of a matrix-type transdermal patch according to the present invention. <Notation of Drawings>
   1: Impenetrable base
   2: Polymer base including effective drug and permeation enhancer
   3: Protection film to be removed before use
FIG. 2 represents the time-course accumulated penetration of buprenorphine hydrochloric acid salt penetrating the skin of a hairless mouse from a hydrogel matrix containing a lipophilic permeation enhancer and from a hydrogel matrix not containing a lipophilic permeation enhancer.
FIG. 3 represents the time-course of the weight change of a matrix caused by leaching of the lipophilic component from a hydrogel matrix that includes an acrylate polymer and a hydrogel matrix that does not includes an acrylate polymer as the compatibilizer.

### Detailed Description of the Invention

Before the present composition and method of use thereof for transdermal delivery of pharmaceutical agents are disclosed and described, it is to be understood that this invention is not limited to the particular configurations, process steps, and materials disclosed herein as such configurations, process steps, and materials may vary somewhat. It is also to be understood that the terminology employed herein is used for the purpose of describing particular embodiments only and is not intended to be limiting since the scope of the present invention will be limited only by the appended claims and equivalents thereof.

It must be noted that, as used in this specification and the appended claims, the singular forms "a," "an," and "the" include plural referents unless the context clearly dictates otherwise. Thus, for example, reference to a composition for delivering "a drug" includes reference to two or more of such drugs, reference to "an adhesive" includes reference to one or more of such adhesives, and reference to "a permeation enhancer" includes reference to two or more of such permeation enhancers.

In describing and claiming the present invention, the following terminology will be used in accordance with the definitions set out below.

As used herein, "effective amount" means an amount of a drug or pharmacologically active agent that is nontoxic but sufficient to provide the desired local or systemic effect and performance at a reasonable benefit/risk ratio attending any medical treatment. An effective amount of a permeation enhancer as used herein means an amount selected so as to provide the desired increase in skin permeability and, correspondingly, the desired depth of penetration, rate of administration, and amount of drug delivered.

As used herein, "transdermal" refers to delivery of a drug through the skin or mucosa and thus includes transmucosal delivery. Similarly, "skin" is meant to include mucosa.

As used herein, "drug," "pharmaceutical agent," "pharmacologically active agent," or any other similar term means any chemical or biological material or compound suitable for transdermal administration by the methods previously known in the art and/ or by the methods taught in the present invention that induces a desired biological or pharmacological effect, which can include but is not limited to (1) having a prophylactic effect on the organism and preventing an undesired biological effect such as preventing an infection, (2) alleviating a condition caused by a disease, for example, alleviating pain or inflammation caused as a result of disease, and/ or (3) either alleviating, reducing, or completely eliminating the disease from the organism. The effect can be local, such as providing for a local anaesthetic effect, or it can be systemic. This invention is not drawn to novel drugs or new classes of active agents. Rather it is limited to the mode of delivery of agents or drugs that exist in the state of the art or that may later be established as active agents and that are suitable for delivery by the present invention. Such substances include broad classes of compounds normally delivered into the body, including through body surfaces and membranes, including skin. In general, this includes but is not limited to: antiinfectives such as antibiotics and antiviral agents; analgesics and analgesic combination; anorexics; antihelminthics; antiarthritics; antiasthmatic agents; anticonvulsants; antidepressants; antidiabetic agents; antidiarrheals; antihistamines; antiinflammatory agents; antimigraine preparations; antinauseants; antineoplastics; antiparkinsonism drugs; antipruritics; antipsychotics; antipyretics; antispasmodics; anticholinergics; sympathomimetics; xanthine derivatives; cardiovascular preparations including potassium and calcium channel blockers, beta-blockers, alpha-blockers, and antiarrhythmics; antihypertensives; diuretics and antidiuretics; vasodilators including general coronary, peripheral, and cerebral; central nervous system stimulants; vasoconstrictors; cough and cold preparations, including decongestants; hormones such as estradiol and other steroids, including corticosteroids; hypnotics; immunosuppressives; muscle relaxants; parasympatholytics; psychostimulants; sedatives; and tranquilizers. By the method of the present invention, ionized drugs can be delivered, as can drugs of either high or low molecular weight.

As used herein, "permeation enhancer," "penetration enhancer," "chemical enhancer," or similar terms refer to compounds and mixtures of compounds that enhance the flux of a drug across the skin. The flux can be increased by changing either the resistance (the diffusion coefficient) or the driving force (the gradient for diffusion).

Chemical enhancers are comprised of two primary categories of components, i.e., cell-envelope disordering compounds and solvents, or binary systems containing both cell-envelope disordering compounds and solvents. The latter are well known in the art, e.g. U.S. Patent Nos. 4,863,970 and 4,537,776, incorporated herein by reference.

Cell envelope disordering compounds are known in the art as being useful in topical pharmaceutical preparations. These compounds are thought to assist in skin penetration by disordering the lipid structure of the cell-envelopes of cells in the stratum corneum. A comprehensive list of these compounds is described in European Patent Application 43,738, published June 13, 1982. Examples of cell envelope disordering compounds that can be used as enhancers, without limitation, include saturated and unsaturated fatty acids and their esters, alcohols, monoglycerides, acetates, diethanolamides, and N,N-dimethylamides such as oleic acid, propyl oleate, isopropyl myristate, glycerol monooleate, glycerol monolaurate, methyl laurate, lauryl alcohol, lauramide diethanolamide, and mixtures thereof. Saturated and unsaturated sorbitan esters, such as sorbitan monooleate and sorbitan monolaurate, can also be used. It is believed that any cell envelope disordering compound is useful for the purposes of this invention.

Suitable solvents include water; and may comprise diols, such as propylene glycol and glycerol; mono-alcohols, such as ethanol, propanol, and higher alcohols; DMSO; dimethylformamide; N,N-dimethylacetamide; 2-pyrrolidone; N-(2-hydroxyethyl) pyrrolidone, N-methylpyrrolidone, 1-dodecylazacycloheptan-2-one and other n-substituted-alkyl-azacycloalkyl-2-ones (azones) and the like.

The present invention is based on the discovery that a stable hydrogel composition can be formulated for transdermal delivery of drugs, wherein an acrylate polymer as defined is used as a compatibilizer which compatibilizes the lipophilic component, i.e. the enhancer, with the hydrophilic polymer base as defined and which renders a uniform composition which is thermodynamically stable.

One embodiment of the present invention is characterized by a transdermal hydrogel composition comprising a hydrophilic polymer base as defined, a drug, a lipophilic permeation enhancer, water as a solvent and a compatibilizer consisting essentially of an acrylate polymer as defined which compatibilizes the lipophilic component, i.e. the enhancer, with the hydrophilic polymer base and which renders a uniform composition which is thermodynamically stable.

In detail, the transdermal hydrogel composition of the present invention comprises:
(1) a hydrophilic polymer base as defined;
(2) an effective amount of a pharmacologically active drug;
(3) a lipophilic permeation enhancer;
(4) an acrylate polymer as defined, which functions as a compatibilizer to enable said lipophilic permeation enhancer to be contained homogeneously and stably in said hydrophilic polymer base; and
(5) water as a solvent.

The hydrophilic polymer base affects the mechanical strength, elasticity and adhesiveness of the transdermal drug delivery system. Suitable hydrophilic polymers of the present invention can comprise one or more members selected from the group consisting of polyvinyl alcohol, polyvinyl pyrrolidone, maleic anhydride/vinyl ether copolymer, gelatin, alginate, hydroxyethyl methacrylate, cargeenane, hydroxyethyl cellulose, silicone rubber, agar, hydroxypropyl cellulose, hydroxypropylmethyl cellulose, methyl cellulose, carboxyvinyl copolymer, polyethylene oxide, polyethylene glycol, polyacryl amide, polyhydroxyethyl methacrylate, polydiorganosiloxane, polyacrylic acid, polyacryl acetate, polyacryl amide and polyvinyl chloride. Preferred hydrophilic polymers are selected from the group consisting of polyvinyl alcohol, polyvinyl pyrrolidone, maleic anhydride/vinyl ether copolymer and hydroxyethyl cellulose.

The adhesiveness to the skin of the hydrophilic polymer base may be controlled through the selection of said polymers. The adhesive property means viscoelasticity which maintains semipermanent adhesion to most of the bases even under very low pressure. The hydrophilic polymer base in the present invention may have enough adhesiveness property by itself or it may function as a pressure sensitive adhesive, or be combined with additional adhesives, plasticizers or other additives.

It is particularly preferred, in terms of adhesion, to use a mixture of polyvinyl alcohol and polyvinyl pyrrolidone as the hydrophilic polymer base, in the range of 2-30wt. % of polyvinyl alcohol and 2-20wt. % of polyvinyl pyrrolidone based on the weight of the transdermal hydrogel composition, and more preferably in the range of 6-15wt.% of polyvinyl alcohol and 4-15wt. % of polyvinyl pyrrolidone.

When the polyvinyl alcohol content is too low, physical properties like the mechanical strength of the matrix worsen. However, if the polyvinyl alcohol content is too high, it is difficult to contain in the matrix the desired amount of drug, enhancer and other fillers due to an increase of solid particles in the base. Furthermore, the flexibility or adhesiveness of the matrix also worsens. When the polyvinyl pyrrolidone content is too low, the adhesiveness of the matrix worsens and the solubility of drugs in the composition decreases because the polymer functions as auxiliary solvent to the drug. If the polyvinyl pyrrolidone content is too high, the manufacturing process becomes difficult due to an increase in viscosity and decrease in the relative content of fillers like permeation enhancers.

Maleic anhydride/vinyl ether copolymer and/or hydroxyethyl cellulose may be used additionally in the mixture of polyvinyl alcohol and polyvinyl pyrrolidone. Preferably, 0.1-15wt.%, more preferably 3-10wt.%, of hydroxyethyl cellulose and/or 0.1-20wt.%, more preferably 0.2-10wt.%, of maleic anhydride/vinyl ether copolymer, are used additionally. Hydroxyethyl cellulose is known to increase the cohesion of the base and to have an effect of decreasing of skin irritation. However, if the hydroxyethyl cellulose content is high, the manufacturing process becomes difficult due to an increase in viscosity and decrease in the relative content of fillers like permeation enhancers. Maleic anhydride/vinyl ether copolymer even in small amount can improve physical properties like adhesion and mechanical strength of the matrix. However, excessive maleic anhydride/vinyl may increase the viscosity or delay the release of the drug.

The permeation enhancer of the present invention functions by various mechanisms, such as by increasing the solubility and diffusion of the drug, changing the water-keeping capacity of the keratotic layer, softening the skin, increasing skin permeability, changing the interfacial state of the skin, or functioning as a hair follicle opener. The permeation enhancer of the present invention may work by more than one mechanism but its fundamental function is to increase permeability of the drug through the skin.

For the permeation enhancer, biphilic or lipophilic permeation enhancers, such as hydrophilic permeation enhancers like C₃-C₄ diols or C₂-C₃ alcohols, C₈-C₁₈ saturated or unsaturated fatty acids, C₈-C₁₈ saturated or unsaturated fatty alcohols, C₂-C₄ alkane diols, C₈-C₁₈ fatty acid esters, fatty alcohol ethers, C₈-C₁₈ saturated or unsaturated fatty acids, esters of C₁-C₄ alcohol or terpene compounds, may be used at less than 65wt. % of the total composition.

Examples of permeation enhancers are polyalcohols like propylene glycol, dipropylene glycol and polyethylene glycol, oils like olive oil, squalene and lanolin, fatty alcohol ethers like cetyl ether and oleyl ether, polyethylene glycol ether which increases the solubility of a drug, fatty acid esters like isopropyl myristate or fatty alcohols like oleyl alcohol that increase the diffusion of a drug, urea or urea derivatives like allantoin that affect the water-keeping capacity of the keratin in skin tissue, polar solvents like dimethyl decyl phosphoxide, methyl octyl sulfoxide, dimethyl lauryl amide, dodecyl pyrrolidone, isosorbitol, dimethyl acetonide, dimethyl sulfoxide, decyl methyl sulfoxide and dimethyl formamide that affect the penetration properties of keratin, keratin softeners like salicylic acid, penetration adjuvants like amino acids, hair follicle openers like benzyl nicotinate and high-molecular fatty acid surfactants like lauryl sulfate which change the status of administered drug and skin surface.

In particular, auxiliary solvents may be added to drugs and drug polymers which are hardly soluble in a hydrophilic system. In the present invention, auxiliary solvents like lecithin, retinal derivatives, tocopherol, dipropylene glycol, triacetin, propylene glycol, saturated or unsaturated fatty acid, mineral oil, silicone fluid and butylbenzyl phthalate may be used.

For another functioning agents, oleic acid, linoleic acid, ascorbic acid, panthenol, butylated hydroxytoluene, tocopherol, tocopheryl acetate, tocopheryl linolate, propyl oleate, isopropyl palmitate, oleamide and polyoxyethylene (4) lauryl ether, polyoxyethylene (2) oleyl ether and polyoxyethylene (10) oleyl ether and polysorbate 20 marketed by ICI Americas trade mark Brij™ 30, 93, 97 and Tween™ 20 may be used additionally.

Though the mechanisms of said permeation enhancer, auxiliary solvent and other functioning agents are different, they may be classified as permeation enhancers because they facilitate skin penetration of drugs through the skin. These permeation enhancers can be classified as hydrophilic, lipophilic or biphilic according to their properties. Most of the permeation enhancers, except for hydrophilic skin-penetration facilitators with hydrophilic groups and few carbon atoms like C₃-C₄ diols or C₂-C₃ alcohols, may be classified as lipophilic or biphilic. Specific examples of permeation enhancers are hydrophilic compounds selected from propylene glycol, glycerol, ethanol, isopropyl alcohol, dimethylsulfoxide and *n*-methyl pyrrolidone; and one or more lipophilic compounds selected from the group consisting of lauryl alcohol, propylene glycol monolaurate, lauroglycol, isopropyl myristate, triacetin, nonanol, oleyl alcohol, linoleyl alcohol, methyl laurate, glycerol monolaurate and glycerol monooleate.

Permeation enhancer to be used in the present invention includes said known compounds, and its specific examples and more details are described in Pharm. Tech., 1990 Sept., 132-136; Pharm. Tech., 1990 Oct., 54-60; Pharm. Tech., 1993 March, 72-98; Pharm. Tech., 1993 Apr., 62-90; and Pharm. Tech., 1993 May, 68-76.

In the polymer hydrogel composition of the present invention, other functioning agents known to facilitate transdermal delivery of drugs may additionally be included.

In the present invention, compatibilizers are used to compatibilize the hydrophobic or lipophilic permeation enhancers in the hydrophilic base composition, comprising a 1:2 ratio of methyl methacrylate and ethyl acrylate expressed by the following Formula (1a) or a copolymer comprising a 1:1 ratio of methacrylic acid and ethyl acrylate expressed by the following Formula (1b), 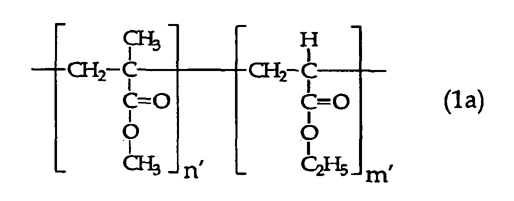 wherein the ratio of n':m' is 1:2, n' is an integer between 200 to 10,000 and m' is an integer between 400 to 20,000. 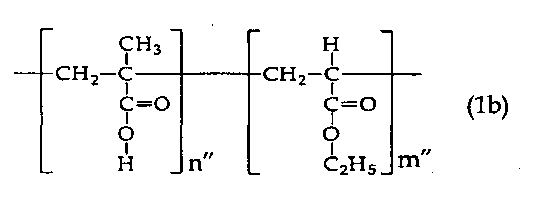 wherein the ratio of n":m" is 1:1, and n" and m" is an integer between 300 to 10,000.

For the compound expressed by Formula (1a), polymethacrylate compounds marketed as Eudragit™ series by Rohm Pharma (Germany) were used; and for the compound expressed by Formula (1b), polymethacrylate compounds marketed as Kollicoat™ series by BASF (USA) were used, in the Examples of the present invention.

The acrylate polymer used as the compatibilizer in the present invention 0.1-10wt.%, preferably 2-8wt. %, of the entire transdermal hydrogel composition. If the compatibilizer content included in the transdermal hydrogel composition is too low, the lipophilic component may be syneresed due to poor compatibility; otherwise if the content is too high, physical properties like the mechanical strength of the matrix may worsen.

The effective drug contained in the transdermal hydrogel composition according to the present invention is pharmacologically or physiologically active for treatment or prevention, and provides a targeted effect when delivered to the body. More specifically, any drug that induces a local or general pharmacological effect for treatment, diagnosis or prevention in plants or animals is regarded to belong to the scope of the present invention. Bioactive drugs like insecticide, pesticides, sunscreens and cosmetics are included in the effective drug list of the present invention. The effective drug is used solely or may be mixed with another effective drug for the prevention, treatment, diagnosis or remedy of diseases or syndromes.

The effective drug is used in a pharmacologically effective amount. This amount means the concentration of a drug that enables a targeted amount of the drug to penetrate the skin with a zero-order penetration rate during the administration period of the drug. This concentration is determined by many parameters including the kind of drug, the administration period of each unit, flow rate of the drug in the system and others. The required amount of effective drug may be determined empirically from the flow rate of the drug and the permeation enhancer used to penetrate the skin. If the required flow rate is determined, a transdermal administration system is designed to have at least the same release rate with the required flow rate. Of course, the surface area of the transdermal administration system affects the release of the drug from the system. The skin-penetration rate means the rate of drug penetrating the skin. This rate may or may not be affected by the release rate of drug from the carrier, as is known in the field of the related art.

The effective drug and mixtures thereof in the present invention can be provided in various forms for optimal drug delivery. Accordingly, the drug may exist as a free-base, acid, salt, ester, or other pharmacologically available derivative forms or molecular complexes.

Various thickeners, fillers or other additives known to be useful for the transdermal drug delivery system may be added to the transdermal hydrogel composition according to the present invention. For example, addition of materials like clay that absorbs water into the composition, is known to increase the adhesive properties without reducing the drug delivery rate. Examples of the clay are kaolinites like vaolinite, anarchsite, dickite and nacrite, montomorillonites like montmorillonite, bentonite, verdelite and montronite, illites/muscobites like illite and glauconite, chlorites and polygorsites like attapulgite, halloysite, metaboloysite, allophane and aluminum silicate clay.

Also, antiseptics can be included in the composition of the present invention. Examples of antiseptic are sodium azide, aminoethyl sulfonic acid, benzoic acid, sodium benzoate, sodium edetate, cetylpyridinum chloride, benzalkonium chloride, benzetonium chloride, sodium sulfate anhydride, isobutyl *p*-oxybenzoate, isopropyl *p*-oxybenzoate and methyl *p*-oxybenzoate.

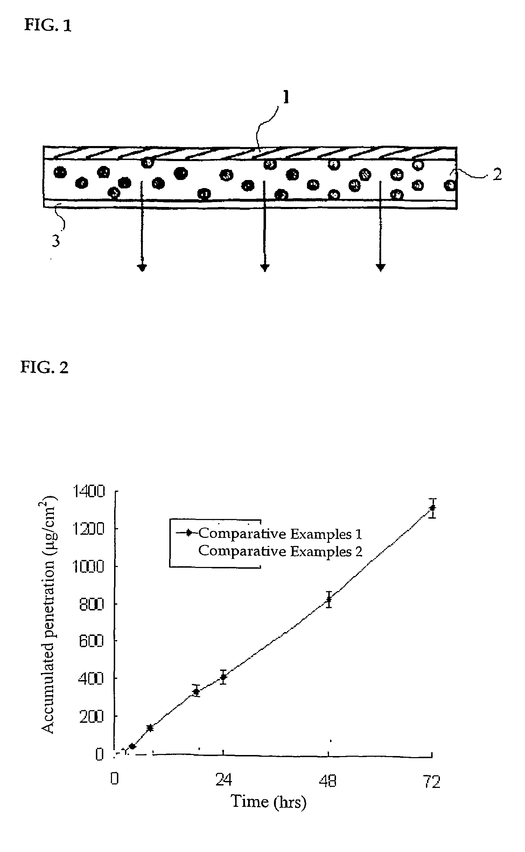

The transdermal hydrogel base composition of the present invention can be used as the adhesive part of any transdermal delivery system or in a matrix type apparatus comprising an adhesive monolayer. FIG. 1 is a typical schematic diagram of a matrix type transdermal administration apparatus comprising an impenetrable base 1, a polymer base 2 including the drug and enhancer, and a protection film 3 to be removed before use. The hydrogel base composition of the present invention can be used as the polymer base 2. Also, the hydrogel base composition of the present invention may be used by adhering it to a common auxiliary base, such as an impenetrable support 1. For the common auxiliary base, a plastic sheet like polyethylene, polypropylene, an ethylene/vinyl acetate copolymer, vinylon, polyester, polyurethane and nylon, a nonwoven fabric like rayon and polyester, a woven fabric like acryl, silk or cotton and a composite layer of these supports may be used.

Hereunder is given a more detailed description of the present invention using examples and comparative examples. However, it should not be construed as limiting the scope of the present invention.

### Comparative Examples 1 & 2

In Comparative Example 1, a transdermal drug delivery matrix including a hydrophobic permeation enhancer was prepared as follows. In a suitable container, a predetermined amount of buprenorphine hydrochloric acid salt, propylene glycol, triacetin, ethanol, lauryl alcohol, glycerol and pure water were added and stirred until the mixture became completely uniform. A predetermined amount of hydroxyethyl cellulose (number-average molecular weight (Mₙ): 250,000), and polyvinyl pyrrolidone was then added, dissolved uniformly then followed by addition of a polyvinyl alcohol (degree of polymerization: 500-2,000) aqueous solution and then mixed uniformly, the mixture was then cooled for about 10hr in a 4-10°C refrigerator.

In Comparative Example 2, a matrix not containing a hydrophobic permeation enhancer was prepared the same way as described in Comparative Example 1 except that no triacetin or lauryl alcohol was added. The composition of Comparative Examples 1 & 2 is shown in Table 1.

**Table 1**

| Composition | Content (wt%) | |
|---|---|---|
| | Comparative Example 1 | Comparative Example 2 |
| Buprenorphine Hydrochloric Acid Salt | 2.0 | 2.0 |
| Propylene Glycol | 19.0 | 24.0 |
| Triacetin (Glycerol Triacetate) | 8.5 | - |
| Ethanol | 14.0 | 12.0 |
| Lauryl Alcohol | 0.5 | - |
| Glycerol | 4.0 | 4.0 |
| Pure Water | 14.0 | 20.0 |
| Hydroxyethyl Cellulose (Mₙ: 250,000) | 4.0 | 4.0 |
| Polyvinyl Pyrrolidine (Collidon 90*) | 10.0 | 10.0 |
| 25% Polyvinyl Alcohol Solution (degree of polymerization: 500-2,000) | 24.0 | 24.0 |
| Total | 100.0 | 100 |

The skin-penetration test of the drug for the matrices prepared from Comparative Examples 1 & 2 were performed as follows. The receptor phase of a Franz Cell was filled with pure water and maintained at 32±0.5°C. The back skin of a male hairless mouse was removed and stabilized for 1hr before the experiment. After cutting the prepared matrix according to the donor cell size, 300µL of sample was taken from the cell after 2, 4, 8, 18, 24, 48 and 72hr, and quantitative analysis was performed using liquid chromatography. The results are shown in FIG. 2 and Table 2.

**Table 2**

| Items | Comparative Example 1 | Comparative Example 2 |
|---|---|---|
| Drug Penetration Rate (Flux; µg/cm²/hr) | 18.49 | 1.76 |
| Retardation Time (hr) | 1.19 | 0.50 |

In Comparative Example 1 wherein a lipophilic permeation enhancer was used, the skin-penetration rate was about 10 times higher than that of Comparative Example 2. Though the retardation time of Comparative Example 2 appeared to be short, the retardation time itself was not of great significance because the penetration rate, and hence the penetration amount, was not as great as is shown in FIG. 2. However, the hydrophobic permeation enhancer (triacetin and lauryl alcohol) used in Comparative Example 1 was syneresed with time due to the low compatibility between the lipophilic component and the hydrophilic base.

The composition of the present invention overcomes this problem and is illustrated by the following examples.

### Example 1

After adding a predetermined amount of buprenorphine hydrochloric acid salt in a suitable container, a predetermined amount of propylene glycol, triacetin, ethanol, lauryl alcohol, glycerol, BASF's Kollicoat MAE 30D™ as acrylate compatibilizer and pure water were added and stirred until the mixture became completely uniform. After adding a predetermined amount of hydroxyethyl cellulose (Mₙ: 250,000) and polyvinyl pyrrolidone herein and dissolving them uniformly, a predetermined amount of a polyvinyl alcohol (degree of polymerization: 500-2,000) aqueous solution was added, mixed uniformly, and then cooled for about 10hr in a 4-10°C refrigerator. The composition of Example 1 is shown in the following Table 3.

**Table 3**

| Composition | Content (wt. %) |
|---|---|
| Buprenorphine Hydrochloric Acid Salt | 2.0 |
| Propylene Glycol | 19.0 |
| Triacetin (Glycerol Triacetate) | 8.5 |
| Ethanol | 14.0 |
| Lauryl Alcohol | 0.5 |
| Glycerol | 4.0 |
| Kollicoat MAE 30D™ | 8.3 |
| Pure Water | 5.7 |
| Hydroxyethyl Cellulose (Mₙ: 250,000) | 4.0 |
| Polyvinyl Pyrrolidine (Collidon 90™) | 10.0 |
| 25% Polyvinyl Alcohol Solution (degree of polymerization: 500-2,000) | 24.0 |
| Total | 100.0 |

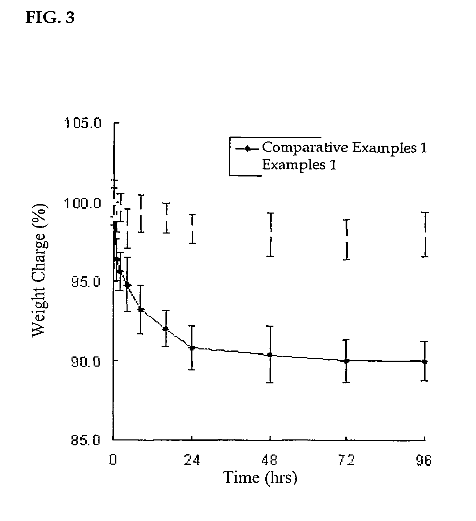

In order to identify the increase of compatibility due to the acrylate polymer included in the present invention, the matrices obtained from Example 1 and Comparative Example 1 were sealed with aluminum foil and kept at room temperature. The syneresed liquid portion was removed with Kim Wipes after 0, 1, 2, 4, 8,16, 24, 48, 72 and 96hr. The weight of the matrices was measured and their change from the initial weight was calculated in %. The results are shown in FIG. 3 and the following Table 4.

**Table 4**

| Time (hr) | Change of Matrix Weight (%, Mean + S.D.) | |
|---|---|---|
| | Comparative Example 1 | Example 1 |
| 1 | 96.4± 0.84 | 99.0± 0.34 |
| 2 | 95.6± .089 | 99.7± 0.21 |
| 4 | 94.8± 0.21 | 98.3± 0.74 |
| 8 | 93.2± 0.16 | 99.3± 0.52 |
| 16 | 92.0± 0.96 | 99.0± 0.14 |
| 24 | 90.8± 0.91 | 98.3± 0.38 |
| 48 | 90.4± 0.38 | 98.0± 0.78 |
| 72 | 90.0± 0.27 | 97.7± 0.34 |
| 96 | 90.0± 0.41 | 98.0± 0.24 |

The weight change of the matrices prepared as in Comparative Example 1 was severe due to syneresation of the thermodynamically unstable composition wherein the lipophilic components were poorly compatible with the hydrophilic polymer base. In addition, volatilization of a volatile solvent like ethanol also contributed to weight loss in some degree. However, in Example 1 wherein an acrylate compatibilizer was used, there was little weight change due to phase separation even after 96hr at room temperature. Therefore, the hydrogel composition of the present invention exhibits superior compatibility and stability of the lipophilic penetration enhancer contained in a hydrophilic polymer base.

Examples 2-14 were performed while adjusting the composition contents of the drug, the hydrogel polymer base and the lipophilic permeation enhancer. Examples 2-6 are not according to the claimed invention.

### Example 2 (Not according to the invention)

| Composition | Content (wt.%) |
|---|---|
| Estradiol | 1.0 |
| Propylene Glycol | 30.0 |
| Polypropylene Glycol Monolaurate | 7.0 |
| Ethanol | 14.0 |
| Cremophore RH 40™ | 0.8 |
| Eudragit NE 30D™ | 6.7 |
| Pure Water | 15.3 |
| Polyvinyl Pyrrolidine (Collidon 30™) | 4.0 |
| Maleic anhydride/Vinyl Ether Copolymer (Gantrez AN 169™) | 21.2 |
| Total | 100.0 |

### Example 3 (Not according to the invention)

| Composition | Content (wt.%) |
|---|---|
| Progesterone | 1.0 |
| Propylene Glycol | 27.0 |
| Lauroglycol (Lacroglyceryl FCC™) | 7.0 |
| Ethanol | 15.0 |
| Cremophore RH 40™ | 6.0 |
| Eudragit NE 30D™ | 6.7 |
| Pure Water | 15.3 |
| Polyvinyl Pyrrolidine (Collidon 30™) | 4.0 |
| Maleic Anhydride Copolymer (Gantrez AN 169™) | 18.0 |
| Total | 100.0 |

### Example 4 (Not according to the invention)

| Composition | Content (wt. %) |
|---|---|
| Albuterol | 2.0 |
| Propylene Glycol | 20.0 |
| Isopropyl Myristate | 6.0 |
| Isopropyl Alcohol | 12.0 |
| Cremophore RH 40™ | 15.0 |
| Eudragit NE 30D™ | 11.7 |
| Pure Water | 11.8 |
| Polyvinyl Pyrrolidine (Collidon 90™) | 4.0 |
| Maleic Anhydride Copolymer (Gantrez AN 169™) | 17.5 |
| Total | 100.0 |

### Example 5 (Not according to the invention)

| Composition | Content (wt.%) |
|---|---|
| Nitroglycerin | 3.0 |
| Propylene Glycol | 15.0 |
| Triacetin (Glycerol Triacetate) | 10.0 |
| Ethanol | 14.0 |
| Lauryl Alcohol | 3.5 |
| Lactic Acid | 2.0 |
| Kollicoat MAE 30D™ | 18.3 |
| Pure Water | 7.2 |
| Polyvinyl Pyrrolidine (Collidon 90™) | 4.0 |
| Maleic Anhydride Copolymer (Gantrez AN 169™) | 23.0 |
| Total | 100.0 |

### Example 6 (Not according to the invention)

| Composition | Content (wt. %) |
|---|---|
| Captopril | 2.0 |
| Propylene Glycol | 12.0 |
| Triacetin (Glycerol Triacetate) | 7.0 |
| Ethanol | 8.0 |
| Lauryl Alcohol | 2.5 |
| Lactic Acid | 2.0 |
| Kollicoat MAE 30D™ | 11.7 |
| Pure Water | 12.8 |
| 25% Polyvinyl Alcohol Aqueous Solution (Degree of Polymerization: 500-2,000) | 32.0 |
| Maleic Anhydride Copolymer (Gantrez AN 169™) | 10.0 |
| Total | 100.0 |

### Example 7

| Composition | Content (wt. %) |
|---|---|
| Pilocarpine | 2.0 |
| Propylene Glycol | 19.0 |
| Triacetin (Glycerol Triacetate) | 9.0 |
| Ethanol | 14.0 |
| Lauryl Alcohol | 0.7 |
| Eudragit NE 30D™ | 11.7 |
| Pure Water | 6.6 |
| Hydroxyethyl Cellulose (Mₙ: 250,000) | 4.0 |
| Polyvinyl Pyrrolidine (Collidon 90™) | 8.0 |
| 20% Polyvinyl Alcohol Aqueous Solution (Degree of Polymerization: 500-2,000) | 25.0 |
| Total | 100.0 |

### Example 8

| Composition | Content (wt.%) |
|---|---|
| Diazepam | 2.0 |
| Propylene Glycol | 11.0 |
| Triacetin (Glycerol Triacetate) | 5.5 |
| Ethanol | 17.5 |
| Lauryl Alcohol | 0.6 |
| Nonanol (Nonyl Alcohol) | 0.6 |
| Eudragit NE 30D™ | 9.3 |
| Pure Water | 14.0 |
| Hydroxyethyl Cellulose (Mₙ: 250,000) | 4.5 |
| Polyvinyl Pyrrolidine (Collidon 90™) | 11.0 |
| 25% Polyvinyl Alcohol Aqueous Solution (Degree of Polymerization: 500-2,000) | 24.0 |
| Total | 100.0 |

### Example 9

| Composition | Content (wt.%) |
|---|---|
| Chlorpromazine | 2.0 |
| Propylene Glycol | 11.0 |
| Triacetin (Glycerol Triacetate) | 4.5 |
| Ethanol | 17.5 |
| Propylene Glycol Monolaurate | 2.0 |
| Nonanol (Nonyl Alcohol) | 0.6 |
| Eudragit NE 30D™ | 8.0 |
| Pure Water | 14.9 |
| Hydroxyethyl Cellulose (Mₙ: 250,000) | 4.5 |
| Polyvinyl Pyrrolidine (Collidon 90™) | 11.0 |
| 25% Polyvinyl Alcohol Aqueous Solution (Degree of Polymerization: 500-2,000) | 24.0 |
| Total | 100.0 |

### Example 10

| Composition | Content (wt.%) |
|---|---|
| Lidocaine | 2.0 |
| Propylene Glycol | 19.5 |
| Triacetin (Glycerol Triacetate) | 5.0 |
| Ethanol | 14.0 |
| Lauryl Alcohol | 0.7 |
| Eudragit NE 30D™ | 12.7 |
| Pure Water | 6.1 |
| Hydroxyethyl Cellulose (Mₙ: 250,000) | 5.0 |
| Polyvinyl Pyrrolidine (Collidon 90™) | 11.0 |
| 25% Polyvinyl Alcohol Aqueous Solution (Degree of Polymerization: 500-2,000) | 20.0 |
| Total | 100.0 |

### Example 11

| Composition | Content (wt%) |
|---|---|
| Buprenorphine | 2.0 |
| Propylene Glycol | 19.5 |
| Triacetin (Glycerol Triacetate) | 9.0 |
| Ethanol | 14.0 |
| Lauryl Alcohol | 0.7 |
| Glycerol | 2.0 |
| Eudragit NE 30D™ | 12.7 |
| Pure Water | 12.1 |
| Hydroxyethyl Cellulose (Mₙ: 250,000) | 5.0 |
| Polyvinyl Pyrrolidine (Collidon 90™) | 11.0 |
| 25% Polyvinyl Alcohol Aqueous Solution (Degree of Polymerization: 500-2,000) | 12.0 |
| Maleic Anhydride Copolymer (Gantrez AN 169™) | 2.0 |
| Total | 100.0 |

### Example 12

| Composition | Content (wt.%) |
|---|---|
| Buprenorphine Hydrochloric Acid Salt | 2.0 |
| Propylene Glycol | 19.5 |
| Triacetin (Glycerol Triacetate) | 9.0 |
| Ethanol | 14.0 |
| Lauryl Alcohol | 0.7 |
| Glycerol | 2.0 |
| Eudragit NE 30D™ | 12.7 |
| Pure Water | 12.1 |
| Hydroxyethyl Cellulose (Mₙ: 250,000) | 5.0 |
| Polyvinyl Pyrrolidine (Collidon 90™) | 11.0 |
| 25% Polyvinyl Alcohol Aqueous Solution (Degree of Polymerization: 500-2,000) | 12.0 |
| Total | 100.0 |

### Example 13

| Composition | Content (wt%) |
|---|---|
| Nicotine | 14.0 |
| Propylene Glycol | 10.0 |
| Triacetin (Glycerol Triacetate) | 4.0 |
| Ethanol | 15.6 |
| Lauryl Alcohol | 1.2 |
| Nonanol (Nonyl Alcohol) | 1.2 |
| Eudragit NE 30D™ | 6.7 |
| Pure Water | 15.3 |
| Hydroxyethyl Cellulose (Mₙ: 250,000) | 4.0 |
| Polyvinyl Pyrrolidine (Collidon 90™) | 8.0 |
| 25% Polyvinyl Alcohol Aqueous Solution (Degree of Polymerization: 500-2,000) | 20.0 |
| Total | 100.0 |

### Example 14

| Composition | Content (wt%) |
|---|---|
| Prostaglandin-E1 | 2.0 |
| Propylene Glycol | 17.0 |
| Triacetin (Glycerol Triacetate) | 8.5 |
| Ethanol | 12.0 |
| Lauryl Alcohol | 0.5 |
| Glycerol | 4.0 |
| Kollicoat MAE 30D™ | 13.3 |
| Pure Water | 4.7 |
| Hydroxyethyl Cellulose (Mₙ: 250,000) | 4.0 |
| Polyvinyl Pyrrolidine (Collidon 90™) | 10.0 |
| 25% Polyvinyl Alcohol Solution (degree of polymerization: 500-2,000) | 24.0 |
| Total | 100 |

As explained above, the transdermal hydrogel composition according to the present invention contains an acrylate polymer as a compatibilizer such that a hydrophilic polymer base and a lipophilic permeation enhancer can be applied simultaneously in order to facilitate the effective local or general skin-penetration of the pharmacologically active drug.

## Claims

1. A transdermal drug delivery composition comprising a hydrophilic polymer base, a drug, a lipophilic permeation enhancer, water as a solvent and a compatibilizer consisting essentially of an acrylate polymer which compatibilizes said lipophilic enhancer with said hydrophilic polymer base and which renders said composition thermodynamically stable, wherein said acrylate polymer is a copolymer comprising a 1:2 ratio of methyl methacrylate and ethyl acrylate expressed by the following Formula (1a), 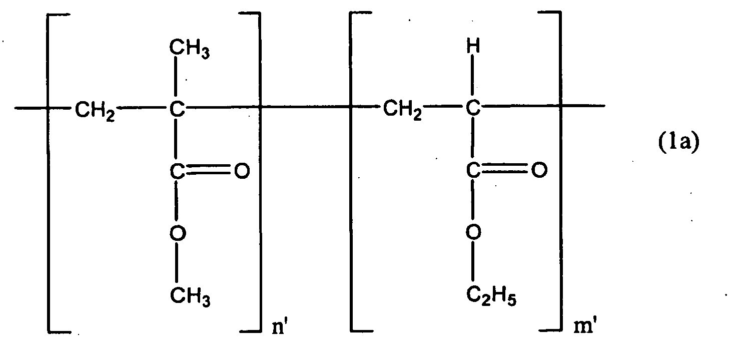 wherein the ratio of n' to m' is 1:2, n' is an integer between 200 to 10,000 and m' is an integer between 400 to 20,000 or a copolymer comprising a 1:1 ratio of methacrylic acid and ethyl acrylate expressed by the following Formula (1b), 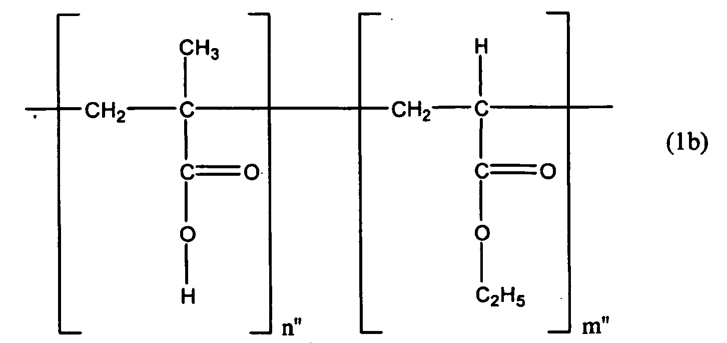 wherein the ratio of n" to m" is 1:1 and n" and m" is an integer between 300 to 10,000, and wherein a mixture of polyvinyl alcohol and polyvinyl pyrrolidone is comprised in the composition as the hydrophylic polymer in the range of 2-30 wt% of polyvinyl alcohol and 2-20 wt% of polyvinyl pyrrolidone based on the weight of the transdermal hydrogel composition.

2. The composition according to claim 1, wherein said acrylate polymer is within the range of 0.1-10wt.% based on the entire composition.

3. The composition according to claim 2, wherein said acrylate polymer is included in the range of 2-8wt.% to the entire composition.

4. The composition according to claim 1, wherein said acrylate polymer has average molecular weight in the range of 50 KD to 5000 KD.

5. The composition according to claim 1, wherein said effective drug is one or more compounds selected from the group consisting of beta-adrenaline activators, beta-adrenaline inhibitors, analgesics, antianginas, antiarrhythmic drugs, antidepressants, antiestrogens, antigonadotrophins, hypotensive drugs, anti-inflammatory drugs, anti-tumor drugs, anti-prostatomegaly drugs, antipsychotics, spasmolytics, antianxiety drugs, bronchodilators, calcium regulators, cardiotonics, dopamine receptors, liver enzyme inducers, estrogens, glucocorticoids, mineral corticoids, monoamine oxidase inhibitors, muscle relaxation drugs, narcotic antagonists, progestogens and peripheral vasodilators.

6. The composition according to claim 5, wherein said effective drug is one or more analgesics selected from the group consisting of buprenorphine and fentanyls like fentanyl, norfentanyl, sufentanyl and alfentanyl.

7. The composition according to claim 1, wherein said hydrophilic polymer further comprises 0.1-15 wt. % of hydroxyethyl cellulose or 0.1-20 wt. % of
maleic anhydride/vinyl ether copolymer.

8. The composition according to claim 1, wherein said permeation enhancer is one or more compounds selected from the group consisting lauryl alcohol, propylene glycol monolaurate, lauroglycol, isopropyl myristate, triacetin, nonanol, oleyl alcohol, linoleyl alcohol, methyl laurate, glycerol monolaurate and glycerol monooleate.

9. The composition according to claim 8, wherein said permeation enhancer is included in the range of 0.1-65 wt. % to the entire composition.

10. A transdermal drug delivery composition comprising a hydrophilic polymer base comprising 2-30 wt. % of polyvinyl alcohol and 2-20 wt. % of polyvinyl pyrrolidone of said hydrophilic polymer base; a drug; a 0.1-65 wt. % of a lipophilic permeation enhancer based on the entire composition; water as a solvent; and 0.1-10 wt. % of a compatibilizer based on the entire composition, said compatibilizer consisting essentially of an acrylate polymer which compatibilizes said lipophilic enhancer with said hydrophilic polymer base and which renders said composition thermodynamically stable, wherein said acrylate polymer is a copolymer comprising a 1:2 ratio of methyl methacrylate and ethyl acrylate expressed by the following Formula (1a), 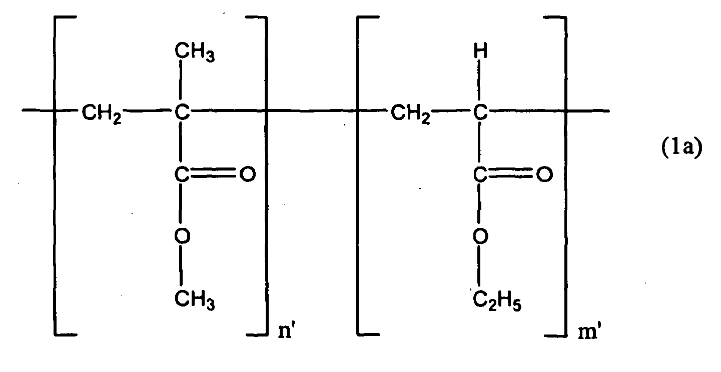 wherein the ratio of n' to m' is 1:2, n' is an integer between 200 to 10,000 and m' is an integer between 400 to 20,000 or a copolymer comprising a 1:1 ratio of methacrylic acid and ethyl acrylate expressed by the following Formula (1b), 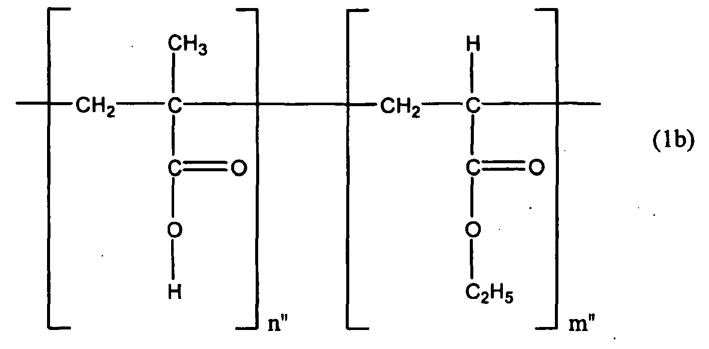 wherein the ratio of n" to m" is 1:1 and n" and m" is an integer between 300 to 10,000.

11. The composition according to claim 10, wherein said acrylate polymer is included in the range of 2-8 wt. % of the entire composition.

12. The composition according to claim 10, wherein said acrylate polymer has average molecular weight in the range of 50 KD to 5000 KD.

13. The composition according to claim 10, wherein said hydrophilic polymer further comprises 0.1-15 wt. % of hydroxyethyl cellulose or 0.1-20 wt. % of maleic anhydride/vinyl ether copolymer.

14. The composition according to claim 10, wherein said permeation enhancer is one or more compounds selected from the group consisting lauryl alcohol, propylene glycol monolaurate, lauroglycol, isopropyl myristate, triacetin, nonanol, oleyl alcohol, linoleyl alcohol, methyl laurate, glycerol monolaurate and glycerol monooleate.

15. A transdermal drug delivery system comprising the transdermal drug delivery composition according to one of the claims 1 to 14.

## Patentansprüche

1. Zusammensetzung zur transdermalen Arzneimittelabgabe, wobei die Zusammensetzung aufweist: eine hydrophile Polymerbasis, ein Arzneimittel, einen lipophilen Permeationsverstärker, Wasser als Lösungsmittel und einen im wesentlichen aus einem Acrylatpolymer bestehenden Kompatibilisierer, der den lipophilen Verstärker mit der hydrophilen Polymerbasis kompatibilisiert und die Zusammensetzung thermodynamisch stabil macht, wobei das Acrylatpolymer ein Copolymer mit Methylmethacrylat und Ethylacrylat in einem Verhältnis von 1:2, ausgedrückt durch die folgende Formel (1a), 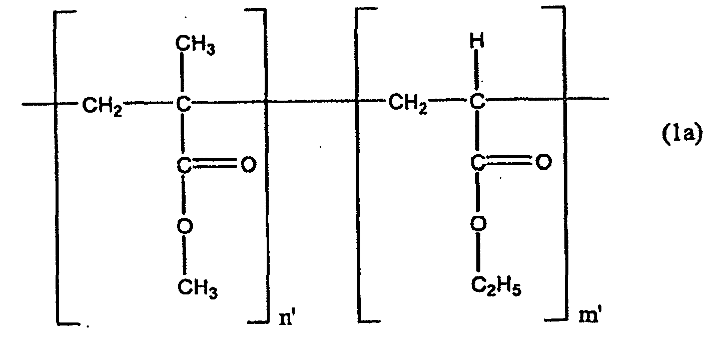 wobei das Verhältnis von n' zu m' gleich 1:2 ist, n' eine ganze Zahl zwischen 200 und 10000 und m' eine ganze Zahl zwischen 400 und 20000 ist, oder ein Copolymer mit Methacrylsäure und Ethylacrylat in einem Verhältnis von 1:1 ist, ausgedrückt durch die folgende Formel (1b), 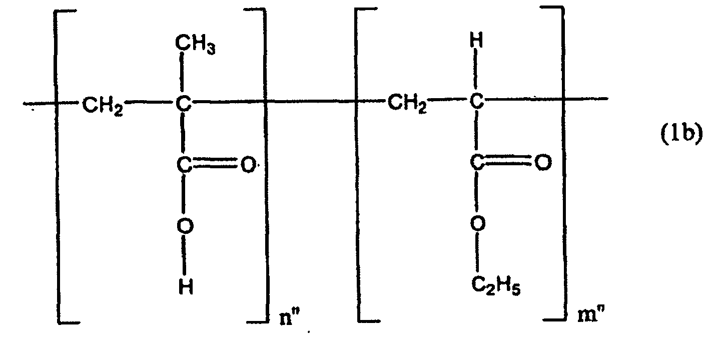 wobei das Verhältnis von n" zu m" gleich 1:1 ist und n" und m" ganze Zahlen zwischen 300 und 10000 sind, und wobei ein Gemisch von Polyvinylalkohol und Polyvinylpyrrolidon in der Zusammensetzung als das hydrophile Polymer in einem Anteil im Bereich von 2-30 Gew.-% Polyvinylalkohol und 2-20 Gew.-% Polyvinylpyrrolidon enthalten ist, bezogen auf das Gewicht der transdermalen Hydrogelzusammensetzung.

2. Zusammensetzung nach Anspruch 1, wobei das Acrylatpolymer im Bereich von 0,1-10 Gew.-% enthalten ist, bezogen auf die Gesamtzusammensetzung.

3. Zusammensetzung nach Anspruch 2, wobei das Acrylatpolymer im Bereich von 2-8 Gew.-% der Gesamtzusammensetzung enthalten ist.

4. Zusammensetzung nach Anspruch 1, wobei das Acrylatpolymer ein mittleres Molekulargewicht im Bereich von 50 kD bis 5000 kD aufweist.

5. Zusammensetzung nach Anspruch 1, wobei der Wirkstoff aus einer oder mehreren Verbindungen besteht, die aus der Gruppe ausgewählt sind, die aus Beta-Adrenalin-Aktivatoren, Beta-Adrenalin-Inhibitoren, Analgetika, Antianginamitteln, Antiarrhythmika, Antidepressiva, Antiöstrogenen, Antigonadotropinen, blutdrucksenkenden Mitteln, entzündungshemmenden Mitteln, Antitumormitteln, Mitteln gegen Prostatomegalie, Antipsychotika, Spasmolytika, Mitteln gegen Angstzustände, Bronchodilatatoren, Calciumregulatoren, Kardiotonika, Dopaminrezeptoren, Leberenzym-Induktoren, Östrogenen, Glukokortikoiden, Mineralkortikoiden, Monoaminoxidase-Inhibitoren, Muskelentspannungsmitteln, Narkotika-Antagonisten, Progestogenen und peripheren Vasodilatatoren besteht.

6. Zusammensetzung nach Anspruch 5, wobei der Wirkstoff aus einem oder mehreren Analgetika besteht, die aus der Gruppe ausgewählt sind, die aus Buprenorphin und Fentanylen wie Fentanyl, Norfentanyl, Sufentanyl und Alfentanyl besteht.

7. Zusammensetzung nach Anspruch 1, wobei das hydrophile Polymer ferner 0,1-15 Gew.-% Hydroxyethylcellulose oder 0,1-20 Gew.-% Maleinsäureanhydrid/Vinylether-Copolymer aufweist.

8. Zusammensetzung nach Anspruch 1, wobei der Permeationsverstärker aus einer oder mehreren Verbindungen besteht, die aus der Gruppe ausgewählt sind, die aus Laurylalkohol, Propylenglycolmonolaurat, Lauroglycol, Isopropylmyristat, Triacetin, Nonanol, Oleylalkohol, Linoleylalkohol, Methyllaurat, Glycerolmonolaurat und Glycerolmonooleat besteht.

9. Zusammensetzung nach Anspruch 8, wobei der Permeationsverstärker in einem Anteil im Bereich von 0,1-65 Gew.-% der Gesamtzusammensetzung enthalten ist.

10. Zusammensetzung zur transdermalen Arzneimittelabgabe, wobei die Zusammensetzung aufweist: eine hydrophile Polymerbasis mit 2-30 Gew.-% Polyvinylalkohol und 2-20 Gew.-% Polyvinylpyrrolidon, bezogen auf die hydrophile Polymerbasis; ein Arzneimittel; 0,1-65 Gew.-% eines lipophilen Permeationsverstärkers, bezogen auf die Gesamtzusammensetzung; Wasser als Lösungsmittel und 0,1-10 Gew.-% eines Kompatibilisierers, bezogen auf die Gesamtzusammensetzung, wobei der Kompatibilisierer im wesentlichen aus einem Acrylatpolymer besteht, das den lipophilen Verstärker mit der hydrophilen Polymerbasis kompatibilisiert und die Zusammensetzung thermodynamisch stabil macht, wobei das Acrylatpolymer ein Copolymer mit Methylmethacrylat und Ethylacrylat in einem Verhältnis von 1:2, ausgedrückt durch die folgende Formel (1a), 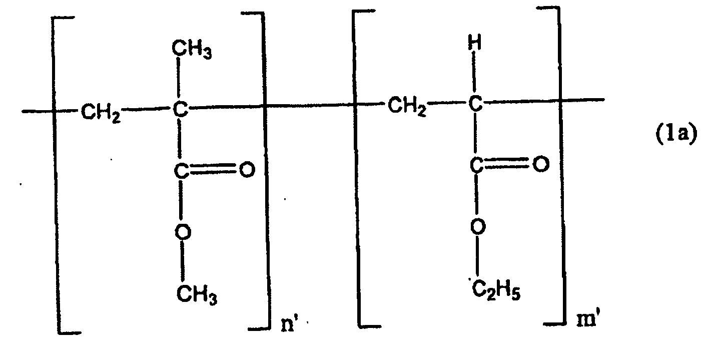 wobei das Verhältnis von n' zu m' gleich 1:2 ist, n' eine ganze Zahl zwischen 200 und 10000 und m' eine ganze Zahl zwischen 400 und 20000 ist, oder ein Copolymer mit Methacrylsäure und Ethylacrylat in einem Verhältnis von 1:1 ist, ausgedrückt durch die folgende Formel (1b), 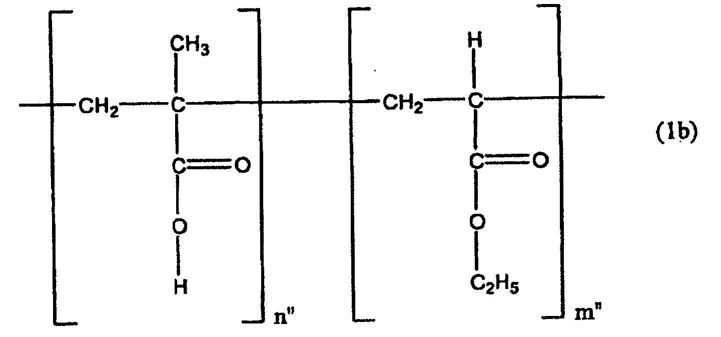 wobei das Verhältnis von n" zu m" gleich 1:1 ist und n" und m" ganze Zahlen zwischen 300 und 10000 sind.

11. Zusammensetzung nach Anspruch 10, wobei das Acrylatpolymer in einem Anteil im Bereich von 2-8 Gew.-% der Gesamtzusammensetzung enthalten ist.

12. Zusammensetzung nach Anspruch 10, wobei das Acrylatpolymer ein mittleres Molekulargewicht im Bereich von 50 kD bis 5000 kD aufweist.

13. Zusammensetzung nach Anspruch 10, wobei das hydrophile Polymer ferner 0,1-15 Gew.-% Hydroxyethylcellulose oder 0,1-20 Gew.-% Maleinsäureanhydrid/Vinylether-Copolymer aufweist.

14. Zusammensetzung nach Anspruch 10, wobei der Permeationsverstärker aus einer oder mehreren Verbindungen besteht, die aus der Gruppe ausgewählt sind, die aus Laurylalkohol, Propylenglycolmonolaurat, Lauroglycol, Isopropylmyristat, Triacetin, Nonanol, Oleylalkohol, Linoleylalkohol, Methyllaurat, Glycerolmonolaurat und Glycerolmonooleat besteht.

15. System zur transdermalen Arzneimittelabgabe, das eine Zusammensetzung zur transdermalen Arzneimittelabgabe nach einem der Ansprüche 1 bis 14 aufweist.

## Revendications

1. Composition de délivrance de médicament transdermique comprenant une base de polymère hydrophile, un médicament, un stimulateur de perméation lipophile, de l'eau en tant que solvant et un agent de compatibilité constitué essentiellement d'un polymère d'acrylate qui rend compatible ledit stimulateur lipophile avec ladite base de polymère hydrophile et qui rend ladite composition thermodynamiquement stable, dans laquelle ledit polymère d'acrylate est un copolymère comprenant un rapport 1:2 de méthacrylate de méthyle et d'acrylate d'éthyle exprimé par la formule (la) suivante, 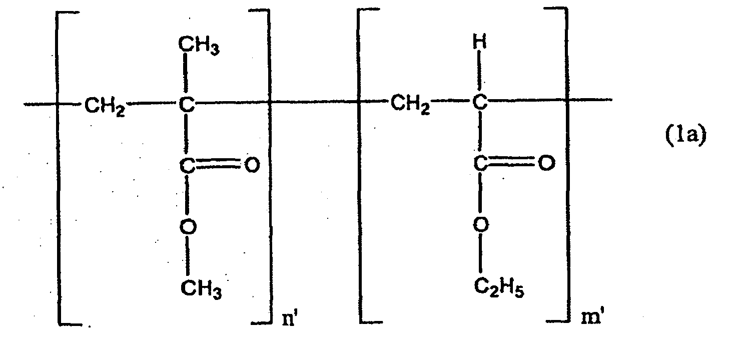 dans laquelle le rapport de n' sur m' est 1:2, n' est un nombre entier entre 200 et 10.000 et m' est un nombre entier entre 400 et 20.000 ou un copolymère comprenant un rapport 1:1 d'acide méthacrylique et d'acrylate d'éthyle exprimé par la formule (1b) suivante, 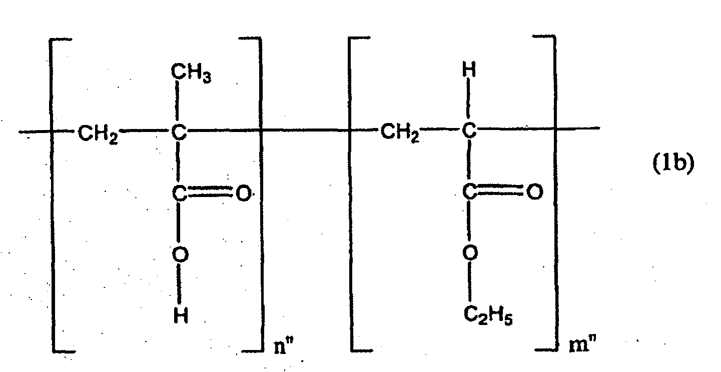 dans laquelle le rapport de n" sur m" est 1:1 1 et n" et m" sont des nombres entiers entre 300 et 10.000, et dans laquelle un mélange d'alcool de polyvinyle et de polyvinylpyrrolidone est compris dans la composition en tant que polymère hydrophile dans l'intervalle de 2-30% en poids d'alcool de polyvinyle et de 2-20% en poids de polyvinylpyrrolidone sur la base du poids de la composition d'hydrogel transdermique.

2. Composition selon la revendication 1, dans laquelle ledit polymère d'acrylate est dans l'intervalle de 0,1-10% en poids sur la base de la composition entière.

3. Composition selon la revendication 2, dans laquelle ledit polymère d'acrylate est inclus dans l'intervalle de 2-8% en poids par rapport à la composition entière.

4. Composition selon la revendication 1, dans laquelle ledit polymère d'acrylate possède un poids moléculaire moyen dans l'intervalle de 50 kD à 5000 kD.

5. Composition selon la revendication 1, dans laquelle ledit médicament efficace est un ou plusieurs composés choisis dans le groupe constitué d'activateurs de bêta-adrénaline, d'inhibiteurs de bêta-adrénaline, d'analgésiques, d'anti-angoreux, de médicaments anti-arythmiques, d'anti-dépresseurs, d'anti-oestrogènes, d'anti-gonadotrophines, de médicaments hypotenseurs, de médicaments anti-inflammatoires, de médicaments anti-tumeur, de médicaments anti-prostatomégalie, d'anti-psychotiques, de spasmolytiques, de médicaments anti-anxiété, de bronchodilatateurs, de régulateurs de calcium, de cardiotoniques, de récepteurs de dopamine, d'inducteurs d'enzyme du foie, d'oestrogènes, de glucocorticoïdes, de corticoïdes minéraux, d'inhibiteurs de monoamine oxydase, de médicaments de relaxation musculaire, d'antagonistes narcotiques, de progestogènes et de vasodilatateurs périphériques.

6. Composition selon la revendication 5, dans laquelle ledit médicament efficace est un ou plusieurs analgésiques choisis dans le groupe constitué de buprénorphine et de fentanyles comme le fentanyle, le norfentanyle, le sufentanyle et l'alfentanyle.

7. Composition selon la revendication 1, dans laquelle ledit polymère hydrophile comprend en outre 0,1-15% en poids d'hydroxyéthylcellulose ou 0,1-20% en poids de copolymère d'anhydride maléique/éther de vinyle.

8. Composition selon la revendication 1, dans laquelle ledit stimulateur de perméation est un ou plusieurs composés choisis dans le groupe constitué d'alcool de lauryle, de monolaurate de propylèneglycol, de lauroglycol, de myristate d'isopropyle, de triacétine, de nonanol, d'alcool d'oléyle, d'alcool de linoléyle, de laurate de méthyle, de monolaurate de glycérol et de monooléate de glycérol.

9. Composition selon la revendication 8, dans laquelle ledit stimulateur de perméation est inclus dans l'intervalle de 0,1-65% en poids par rapport à la composition entière.

10. Composition de délivrance de médicament transdermique comprenant une base de polymère hydrophile comprenant 2-30% en poids d'alcool de polyvinyle et 2-20% en poids de polyvinylpyrrolidone de ladite base de polymère hydrophile; un médicament; 0,1-65% en poids d'un stimulateur de perméation lipophile sur la base de la composition entière; de l'eau en tant que solvant; et 0,1-10% en poids d'un agent de compatibilité sur la base de la composition entière, ledit agent de compatibilité étant constitué essentiellement d'un polymère d'acrylate qui rend compatible ledit stimulateur lipophile avec ladite base de polymère hydrophile et qui rend ladite composition thermodynamiquement stable, dans laquelle ledit polymère d'acrylate est un copolymère comprenant un rapport 1:2 de méthacrylate de méthyle et d'acrylate d'éthyle exprimé par la formule (la) suivante, 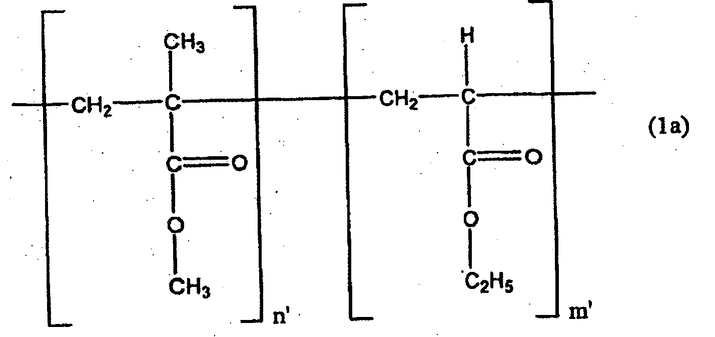 dans laquelle le rapport de n' sur m' est 1:2, n' est un nombre entier entre 200 et 10.000 et m' est un nombre entier entre 400 et 20.000 ou un copolymère comprenant un rapport 1:1 d'acide méthacrylique et d'acrylate d'éthyle exprimé par la formule (1b) suivante, 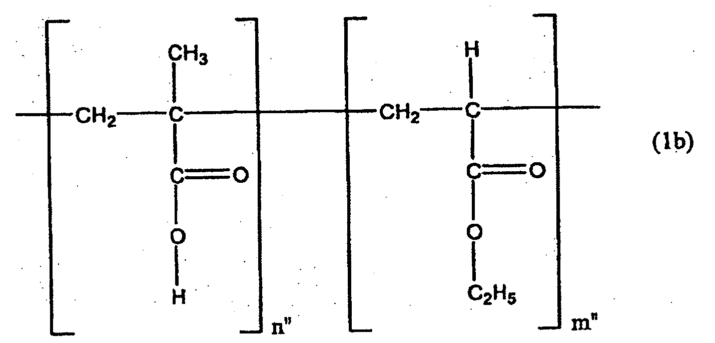 dans laquelle le rapport de n" sur m" est 1:1 1 et n" et m" sont des nombres entiers entre 300 et 10.000.

11. Composition selon la revendication 10, dans laquelle ledit polymère d'acrylate est inclus dans l'intervalle de 2-8% en poids de la composition entière.

12. Composition selon la revendication 10, dans laquelle ledit polymère d'acrylate possède un poids moléculaire moyen dans l'intervalle de 50 kG à 5000 kD.

13. Composition selon la revendication 10, dans laquelle ledit polymère hydrophile comprend en outre 0,1-15% en poids d'hydroxyéthylcellulose ou 0,1-20% en poids de copolymère d'anhydride maléique/éther de vinyle.

14. Composition selon la revendication 10, dans laquelle ledit stimulateur de perméation est un ou plusieurs composés choisis dans le groupe constitué d'alcool de lauryle, de monolaurate de propylèneglycol, de lauroglycol, de myristate d'isopropyle, de triacétine, de nonanol, d'alcool d'oléyle, d'alcool de linoléyle, de laurate de méthyle, de monolaurate de glycérol et de monooléate de glycérol.

15. Composition de délivrance de médicament transdermique comprenant la composition de délivrance de médicament transdermique selon l'une quelconque des revendications 1 à 14.
